# EUROPEAN PATENT APPLICATION

(11) **EP 2 807 994 A2**
(43) Date of publication of application: **03.12.2014**
(21) Application number: 14164191.0
(22) Date of filing: 10.04.2014
(51) Int. Cl.: A61F 13/02, A61F 13/00

(54) **Wound dressing formed with a two-sided adhesive band**

(30) Priority: 30.05.2013 US 201313905611
(71) Applicant: Flextronics International USA Inc., San Jose, CA 95002 (US)
(72) Inventor: Nelson, Andrew, Dallas, TX Texas 75220 (US)
(74) Representative: Rupprecht, Kay

(57) **Abstract**

A wound dressing and method of forming the same. In one embodiment, the wound dressing includes a two-sided adhesive band conformable to a surface of a person. The wound dressing also includes a removable bandage configured to attach to an upper surface of and seat over the two-sided adhesive band, thereby forming a substantially closed structure for application over a wound on the surface of the person.

## Description

### TECHNICAL FIELD

The present invention is directed, in general, to wound dressings and, more specifically, to a wound dressing formed with a two-sided adhesive band and method of forming the same.

### BACKGROUND

In medical and other applications, a wound dressing is generally employed to provide an outer physical layer of protection for a wound such as a burned area of skin or an open or closed wound resulting from a surgical incision or from an accident. Conventional approaches to protecting a wound typically involve an application of a wound dressing formed with an adhesive layer applied to a flexible film such as a plastic strip. The wound dressing includes a patch of a soft and absorbent wound-contacting component such as a gauze or cloth material that exhibits a tendency to form a bond over time with the wound over the area of application.

An element of wound healing generally requires frequent cleansing of the wound and adjacent skin areas, at least during the initial stages of wound healing. An issue that should be carefully addressed is dealing with exudate from the wound, which can penetrate and harden in the wound-contacting portion of the wound dressing. However, the delicate nature of the wound and adjacent areas of skin often induce pain and further injury upon removal of the wound dressing as a result of strong attachment of the adhesive layer on the wound dressing to adjacent areas of skin, particularly to body hair adjacent the wound. The wound-contacting component of the wound dressing also bonds over time to the hardened wound exudate, which necessitates frequent cleaning and replacement of the wound dressing.

Thus, conventional approaches to forming a wound dressing that enable personnel to provide effective wound care raise questions of patient comfort and safety. Accordingly, what is needed in the art is a process and method to form a wound dressing that avoids disadvantages of current approaches.

### SUMMARY OF THE INVENTION

Technical advantages are generally achieved, by advantageous embodiments of the present invention, including a wound dressing and method of forming the same. In one embodiment, the wound dressing includes a two-sided adhesive band conformable to a surface of a person, and a removable bandage configured to attach to an upper surface of and seat over the two-sided adhesive band, thereby forming a substantially closed structure for application over a wound on the surface of the person.

The foregoing has outlined rather broadly the features and technical advantages of the present invention in order that the detailed description of the invention that follows may be better understood. Additional features and advantages of the invention will be described hereinafter, which form the subject of the claims of the invention. It should be appreciated by those skilled in the art that the conception and specific embodiment disclosed may be readily utilized as a basis for modifying or designing other structures or processes for carrying out the same purposes of the present invention. It should also be realized by those skilled in the art that such equivalent constructions do not depart from the spirit and scope of the invention as set forth in the appended claims.

### BRIEF DESCRIPTION OF THE DRAWINGS

For a more complete understanding of the present invention, reference is now made to the following descriptions taken in conjunction with the accompanying drawings, in which:
FIGURE 1 illustrates a view of an embodiment of a wound dressing;
FIGUREs 2 and 3 illustrate views of an application of an embodiment of a wound dressing to a person;
FIGUREs 4 and 5 illustrate views of embodiments of a wound dressing in a closed, wearing position;
FIGURE 6 illustrates a view of a removal of an embodiment of a removable bandage from a two-sided adhesive band of a wound dressing; and
FIGUREs 7 and 8 illustrate views of another embodiment of a wound dressing in an open and closed position, respectively.

Corresponding numerals and symbols in the different figures generally refer to corresponding parts unless otherwise indicated, and may not be redescribed in the interest of brevity after the first instance. The FIGUREs are drawn to illustrate the relevant aspects of exemplary embodiments.

### DETAILED DESCRIPTION OF ILLUSTRATIVE EMBODIMENTS

The making and using of the present exemplary embodiments are discussed in detail below. It should be appreciated, however, that the present invention provides many applicable inventive concepts that can be embodied in a wide variety of specific contexts. The specific embodiments discussed are merely illustrative of specific ways to make and use the invention, and do not limit the scope of the invention.

The present invention will be described with respect to exemplary embodiments in a specific context, namely, a wound dressing for application after an injury or surgical procedure. The wound dressing is formed with a two-sided adhesive band that is conformable to a surface of a person and a removable bandage. While the principles of the present invention will be described in the environment of a medical application, any application that may benefit from a wound dressing is well within the broad scope of the present invention.

It is known to make wound dressings for use on patients with high-risk wounds such as highly exuding wounds. Such wounds can be acute or chronic, or can result from a surgical incision, an accident, a skin graft, etc. Dressings for such wounds manage wound exudate in a variety of ways. For instance, some dressings, particularly those based on foam, manage wound exudate by absorbing the wound exudate and allowing moisture absorbed by the dressing to evaporate through the backing or top of the dressing. Such wound dressings are often not designed to absorb and retain the exudate, but to absorb and expel the exudate by moisture vapor transmission so that the wound dressing maximizes a level of exudate handled within the limitations of the design. A disadvantage of such wound dressings is that the lateral spread of the exudate across the dressing may not be contained because of the nature of the open structure of foam, and this can cause normal skin surrounding the wound to become macerated or otherwise adversely affected as the whole of the dressing surface becomes saturated.

A person with such an acute or chronic wound may use a negative pressure wound therapy ("NPWT") pump that requires frequent and often painful removal and replacement of bandages. Even though use of an NPWT pump decreases the frequency of dressing changes compared to use of a conventional dressing, trauma for the patient and wound still remain when a time comes for a dressing change.

As introduced herein, a wound dressing is formed with a two-layer bandage. A first layer of the two-layer bandage is formed with a two-sided adhesive band. A lower side of the two-sided adhesive band is adhered directly around the wound area. The lower side has a high-tack biocompatible adhesive that is placed on/against a surface of a person. The upper side of the two-sided adhesive band has a feature that allows a bandage that can be coupled thereto to be removed easily and replaced with fresh gauze. The feature on the upper side could be formed of a closure similar to that of a zip-lock bag, a low-stick adhesive, or even a low-tack hook-and-loop structure similar to Velcro®. The two-sided adhesive band can be removed when the wound is partially or fully healed, or for additional cleaning of the wound area. A second layer of the wound dressing is a replaceable/disposable portion. It is configured to stay in situ when combined with the surface-level, two-sided adhesive band. For instance, the second layer may contain a port for connecting to a negative-pressure wound therapy pump.

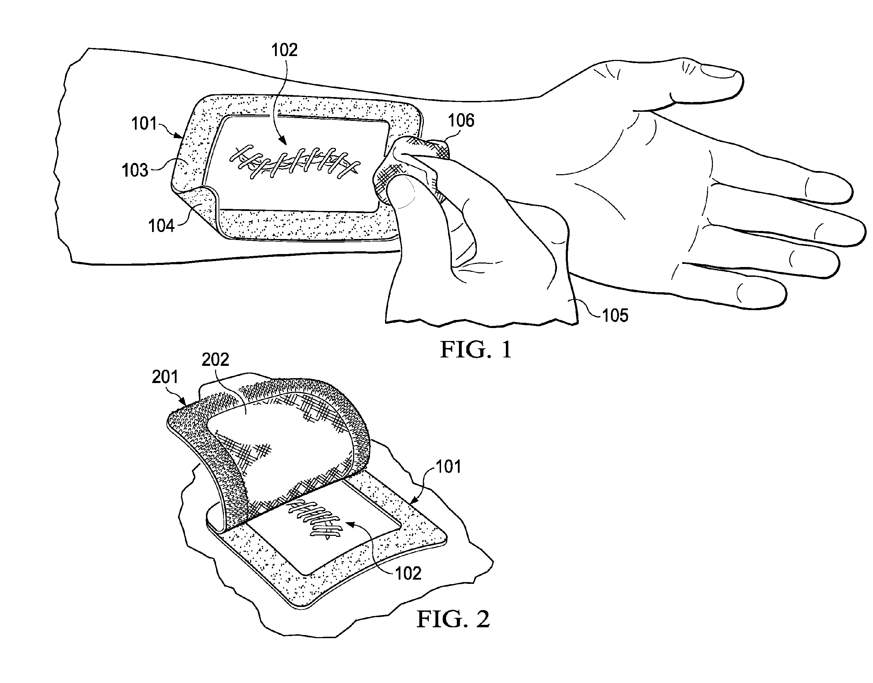

The wound dressing enables a care-giver to easily remove a saturated bandage from a wound area in a short period of time, clean the affected skin, and replace the bandage with a clean gauze without causing excessive trauma to either the patient or the wound. Benefits of the wound dressing include ease of use, reduction of wound trauma, wound cleaning speed, reduced nurse time, painless bandage replacement, patient comfort, and improved patient quality of life. Referring initially to FIGURE 1, illustrated is a view of an embodiment of a two-sided adhesive band 101 that provides a clean semi-permanent base for a wound dressing. The semi-permanent base is flexible and conformable to a surface of a person such as exposed skin of a patient's body. The term semi-permanent describes a base that is infrequently removed from a patient relative to a removable flexible bandage that is applied over the semi-permanent band. The two-sided adhesive band 101 is formed with a light adhesive layer on an upper surface 103 and a stronger adhesive layer on a lower surface 104 that is bondable to the skin, and surrounds a wound or incision 102. FIGURE 1 also illustrates a hand 105 of a care giver employing a gauze-like material 106 to clean exudate about the two-sided adhesive band 101 caused by the wound or incision 102.

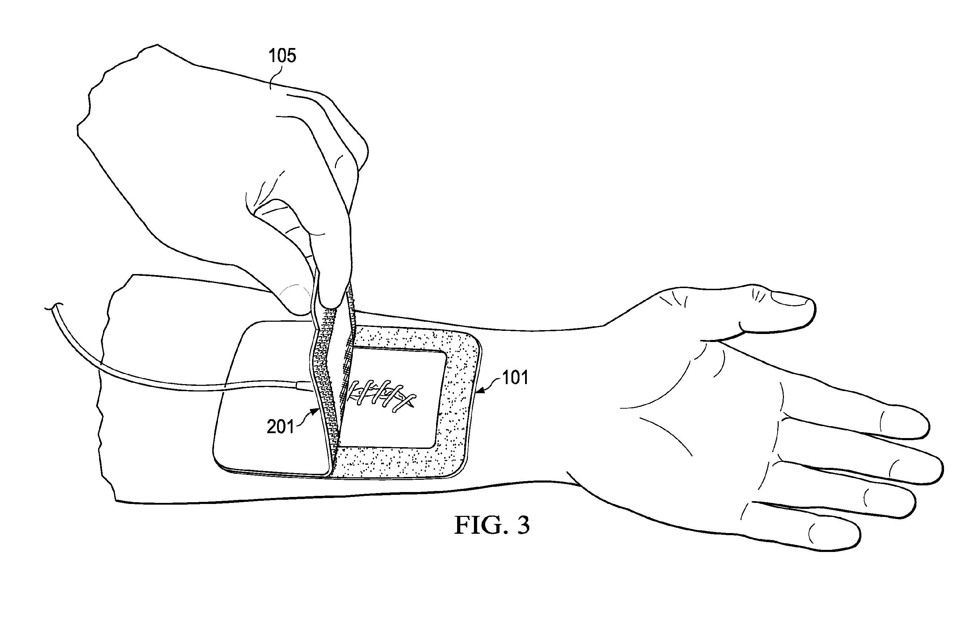

Referring now to FIGUREs 2 and 3, illustrated are views of an application of an embodiment of a wound dressing to a person. The wound dressing includes a two-sided adhesive band 101 applied to a surface of a person such as exposed skin of a patient's body surrounding the wound or incision 102. A removable bandage such as a removable flexible bandage 201 with a light tack adhesive layer is applied over the semi-permanent, flexible two-sided adhesive band 101. For purposes of illustration, the hand 105 of the care giver is shown partially removing the removable flexible bandage 201 from the two-sided adhesive band 101. A wound-contacting layer 202 is formed on an interior/lower surface of the removable flexible bandage 201 with a water-permeable fabric and is applied over and in contact with the two-sided adhesive band 101, and over the wound or incision 102. Thus, the upper surface of the two-sided adhesive band 101 forms a hook or loop structure coupled to a complementary loop or hook structure on the removable flexible bandage 201 akin to Velcro®.

The wound-contacting layer 202 can be formed with a water-permeable fabric that can be infused (or combined) into an intermediate layer thereof with a mixture including an infection resisting agent and a moisture-absorbing agent. The wound-contacting layer 202 can be, without limitation, a bio-compatible, biodegradable macromolecular water-absorbent polymeric material, a macromolecular water-absorbent hybrid material ("WAHM"), a natural or man-made biocompatible, non-irritating hemostatic agent, or gauze.

Honey can be used as an antimicrobial agent for the wound-contacting layer 202. Antimicrobial properties of honey have been reported and honey of various geographical origins is commonly used as a base for ointments. Honey as an alternative to silver-based compounds such as silver sulfadiazine or silver nanomaterials has been successfully applied in surgical dressings for open wounds and burns to avoid septic infections as an infection resisting agent. Other antimicrobial agents can be used including, without limitation, other natural or man-made antimicrobial agents, e.g., Neosporin™.

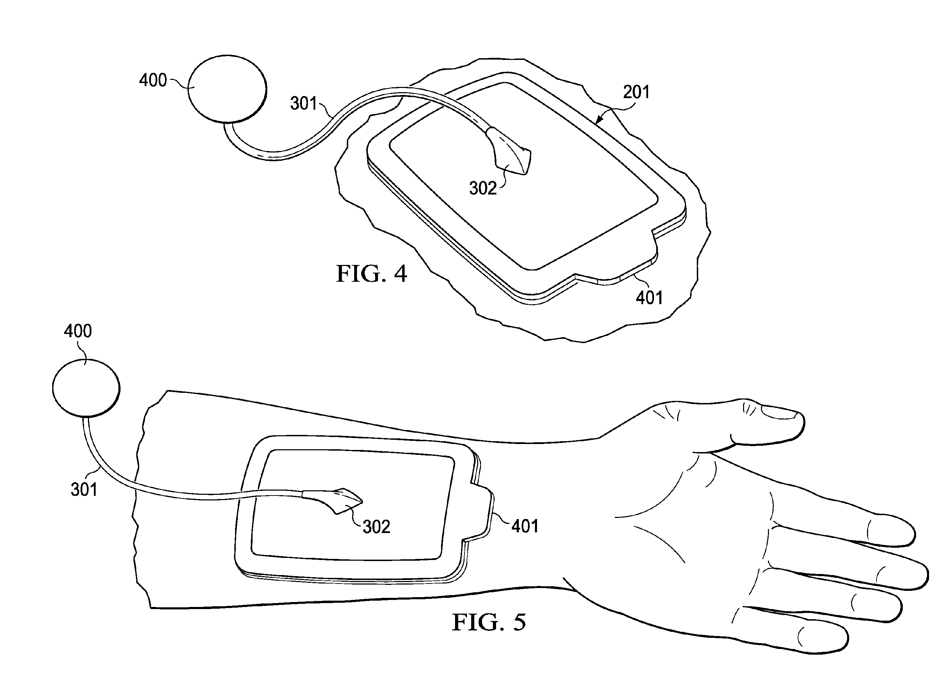

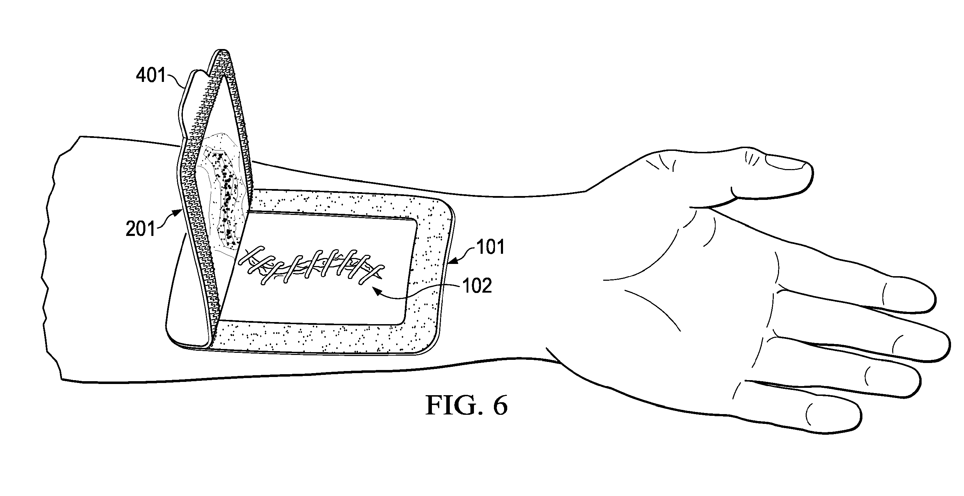

Turning now to FIGUREs 4 and 5, illustrated are views of embodiments of a wound dressing in a closed, wearing position. The wound dressing is coupled by a tube such as a disposable tube 301 to an air pump 400 for creating a small, negative pressure relative to the surrounding atmospheric pressure between a removable flexible bandage 201 and a surface of a person such as exposed skin of a patient's body. The disposable tube 301 is coupled to the wound dressing with a tube connector 302 formed or otherwise installed on a surface of the removable flexible bandage 201. The removable flexible bandage 201 also includes a tab 401 to assist in removing the removable flexible bandage 201 from the two-sided adhesive band 101 as illustrated with respect to FIGURE 6.

Turning now to FIGURE 6, illustrated is a view of a removal of a removable flexible bandage 201 from a two-sided adhesive band 101 of a wound dressing. The removable flexible bandage 201 is lightly bonded to the two-sided adhesive band 101, which facilitates easy removal therefrom (in accordance with a tab 401), exposing the wound or incision 102 with minimal trauma and discomfort to the person. The removable flexible bandage 201 can thereby be easily replaced with a fresh sterile removable flexible bandage 201.

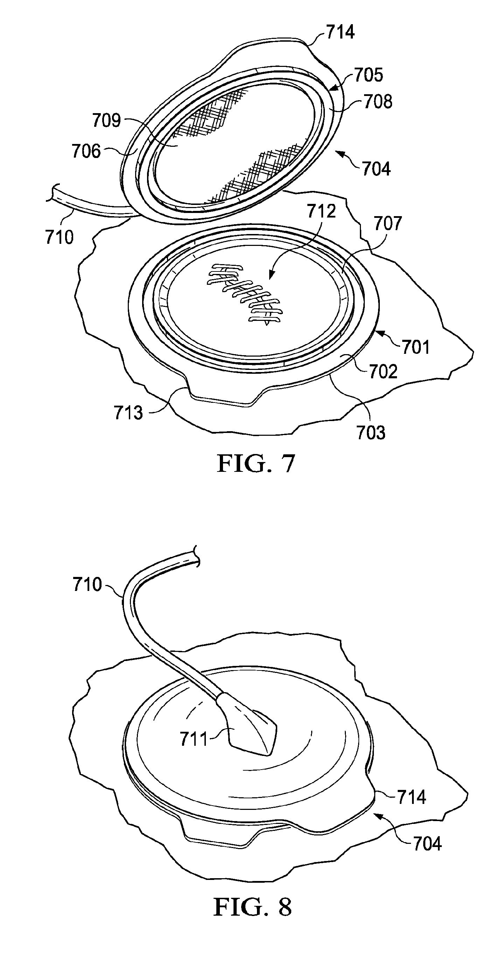

Turning now to FIGUREs 7 and 8, illustrated are views of an embodiment of a wound dressing in an open and closed position, respectively. In this embodiment, a bandage base such as a semi-permanent bandage base 701 includes a first ring such as a female mating ring 707 on an upper surface 702 thereof and with a lower surface 703 applied via an adhesive layer to a surface of a person such as an exposed surface of a patient's skin surrounding a wound or incision 712. The bandage base 701 is semi-rigid, adhesive backed, and comfortable to the patient's skin. The bandage base 701 includes a tab 713 without the adhesive layer for easier removal from the surface of the person.

A removable bandage such as a semi-rigid, removable, flexible bandage 704 includes a second ring such as a round, male mounting ring 705 on a lower surface 706 thereof to eliminate misaligned application of the wound dressing. The female mating ring 707 formed with a groove mates with a complementary structure such as a tongue/protrusion 708 of the male mounting ring 705. Thus, the male mounting ring 705 is configured to mate removably with the female mating ring 707 to form a substantially closed structure for application over the wound or incision 712. The male mounting ring 705 is also formed with a tab 714 to assist in removing the flexible bandage 704 from the two-sided adhesive band/bandage base 701. The adhesive layer on the lower surface 703 of the bandage base 701 bonds more strongly to the patient's skin than the male mounting ring 705 mates with the female mating ring 707. A wound-contacting layer 709 (e.g., a water-permeable fabric) lines an outer layer of the removable flexible bandage 704 and is coupled to the outer layer with a light adhesive tack. The wound-contacting layer 709 may include features as described above. A tube such as a disposable tube 710 and tube connector 711 (akin to the disposable tube 301 and tube connector 302 described above) coupled to the removable flexible bandage 704 are illustrated in FIGURE 8 as the wound dressing is in a closed position.

Those skilled in the art should understand that the previously described embodiments of a wound dressing and related methods of forming the same are submitted for illustrative purposes only. A wound dressing as described hereinabove may also be applied to other applications such as, without limitation, in a veterinary environment. Thus, even though the embodiments discussed herein are illustrated with respect to a person, any creature such as a dog may benefit from the principles as described herein.

Also, although the present invention and its advantages have been described in detail, it should be understood that various changes, substitutions and alterations can be made herein without departing from the spirit and scope of the invention as defined by the appended claims. For example, many of the processes discussed above can be implemented in different methodologies and replaced by other processes, or a combination thereof.

Moreover, the scope of the present application is not intended to be limited to the particular embodiments of the process, machine, manufacture, composition of matter, means, methods, and steps described in the specification. As one of ordinary skill in the art will readily appreciate from the disclosure of the present invention, processes, machines, manufacture, compositions of matter, means, methods, or steps, presently existing or later to be developed, that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A wound dressing, comprising:
a two-sided adhesive band conformable to a surface of a person; and
a removable bandage configured to attach to an upper surface of and seat over said two-sided adhesive band, thereby forming a substantially closed structure for application over a wound on said surface of said person.

2. The wound dressing as recited in Claim 1 further comprising a tube connector formed on said removable bandage configured to enable a reduced pressure relative to an atmospheric pressure to be produced under said wound dressing by a pump coupled to said tube connector via a tube.

3. The wound dressing as recited in Claim 1 wherein said two-sided adhesive band comprises a light adhesive layer on said upper surface bondable to said removable bandage and a stronger adhesive layer on a lower surface bondable to said surface of said person.

4. The wound dressing as recited in Claim 1 wherein said removable bandage includes a tab configured to assist in removing said removable bandage from said two-sided adhesive band.

5. The wound dressing as recited in Claim 1 wherein said two-sided adhesive band is formed with an adhesive layer on a lower surface bondable to said surface of said patient's body, and a hook or loop structure on said upper surface couplable to a complementary loop or hook structure on said removable bandage.

6. The wound dressing as recited in Claim 1 wherein said removable bandage includes a wound-contacting layer on a lower surface comprising a water-permeable fabric.

7. The wound dressing as recited in Claim 6 wherein said water-permeable fabric is infused with a mixture comprising an infection resisting agent and a moisture-absorbing agent.

8. The wound dressing as recited in Claim 7 wherein said infection resisting agent comprises honey.

9. The wound dressing as recited in Claim 7 wherein said moisture-absorbing agent comprises gauze.

10. The wound dressing as recited in Claim 7 wherein said mixture is combined into an intermediate layer of said water-permeable fabric.

11. A wound dressing, comprising:
an bandage base conformable to a surface of a person comprising a first ring formed on an upper surface thereof; and
a removable bandage with a second ring formed on a lower surface thereof, said second ring configured to mate removably with said first ring to form a substantially closed structure for application over a wound on said surface of said person.

12. The wound dressing as recited in Claim 11 further comprising a tube connector formed on said removable bandage configured to enable a reduced pressure relative to atmospheric pressure to be produced under said wound dressing by a pump coupled to said tube connector via a pump.

13. The wound dressing as recited in Claim 11 wherein said first ring includes a grove configured to mate with a protrusion of said second ring.

14. The wound dressing as recited in Claim 11 wherein said bandage base further comprises an adhesive layer on a lower surface thereof bondable to said surface of said person.

15. The wound dressing as recited in Claim 14 wherein said adhesive layer bonds more strongly to said surface of said person than said second ring mates with said first ring.

16. The wound dressing as recited in Claim 11 wherein said removable bandage includes a wound-contacting layer comprising a water-permeable fabric.

17. The wound dressing as recited in Claim 16 wherein said water-permeable fabric is infused with a mixture comprising an infection-resisting agent and a moisture-absorbing agent.

18. The wound dressing as recited in Claim 17 wherein said infection resisting agent comprises honey.

19. The wound dressing as recited in Claim 17 wherein said moisture-absorbing agent comprises gauze.

20. The wound dressing as recited in Claim 17 wherein said mixture is combined into an intermediate layer of said water-permeable fabric.
